# EUROPEAN PATENT APPLICATION

(11) **EP 4 491 174 A1**
(43) Date of publication of application: **15.01.2025**
(21) Application number: 24169842.2
(22) Date of filing: 12.04.2024
(51) Int. Cl.: A61K 9/16, A61K 31/606, A61K 9/00

(54) **MESALAZINE EXTENDED RELEASE COMPOSITION AND PROCESS FOR PREPARATION THEREOF**

(30) Priority: 02.05.2023 IN 202311031338
(71) Applicant: Athena Pharmaceutiques SAS, 78430 Louveciennes (FR)
(72) Inventor: Chaudhari, Mahendra Baliram, 400602 Thane Maharashtra (IN); Chandwani, Omprakash Doulatram, 421301 Maharashtra (IN); Nehete, Nitin Pandharinath, 421201 Maharashtra (IN); Chaudhari, Amol Yuvraj, 421202 Maharashtra (IN)
(74) Representative: Ibarra Garcia, Isabel

(57) **Abstract**

The present invention relates to a mesalazine extended release composition and a process for preparation thereof. The mesalazine extended release composition comprises: about 55 to about 65 percent by weight of mesalazine; about 26 to about 30 percent by weight of sugar spheres; about 4 to about 6 percent by weight of a binder; about 0.3 to about 0.75 percent by weight of a first plasticizer; about 3 to about 7 percent by weight of a release controlling agent; about 2 to about 5 percent by weight of a pore forming agent; about 0.4 to about 1.5 percent by weight of a second plasticizer; and about 1 to about 3 percent by weight of a lubricant. The binder is a water-miscible aqueous dispersion. The release controlling agent functions independently of pH.

## Description

### FIELD OF THE INVENTION

The present invention relates to an extended release pharmaceutical formulation comprising mesalazine. In particular, the present invention relates to a particulate pharmaceutical formulation comprising active drug mesalazine for oral administration as well as a method for producing it for sachet formulation.

### BACKGROUND OF THE INVENTION

Mesalazine, also known as mesalamine is chemically (5-aminosalicylic acid. Chemical structure of mesalazine is as followed.

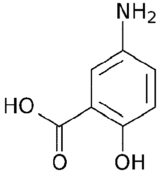

Mesalazine is used to treat ulcerative colitis (a condition in which part or all the linings of the colon [large intestine] are swollen or worn away). Mesalazine delayed-release tablets and controlled-release capsules may be used to treat ulcerative colitis that affects any part of the colon. Conventional mesalazine pellets have been available for a long time; however, the process for manufacturing of these conventional pellets is not efficiently reproducible and requires a number of machines for conducting the different manufacturing steps which results in a slower process. Also, the layering process to prepare the conventional mesalamine pellets does not provide for a high drug content suspension.

Accordingly, there is a need for sachet dosage having composition that has improved extended release, requires less manufacturing time, uses minimal ingredients, has improved bioavailability and process thereof to manufacture mesalazine extended release composition that is faster, simple, easier, robust, automated, and reproducible.

### SUMMARY OF THE INVENTION

To achieve the foregoing and other objects and needs, the present invention provides a mesalazine extended release composition that has improved extended release, requires less manufacturing time, uses minimal ingredients, and has improved bioavailability. Further, the present invention provides a process for preparation of mesalazine extended release composition that is faster, simple, easier, robust, automated, and reproducible wherein all the steps of preparation of the mesalazine extended release composition can be performed using minimum equipments.

In an embodiment, the present invention relates to mesalazine extended release composition comprising: about 55 to about 65 percent by weight of mesalazine; about 26 to about 30 percent by weight of sugar spheres; about 4 to about 6 percent by weight of a binder; about 0.3 to about 0.75 percent by weight of a first plasticizer; about 3 to about 7 percent by weight of a release controlling agent; about 2 to about 5 percent by weight of a pore forming agent; about 0.4 to about 1.5 percent by weight of a second plasticizer; and about 1 to about 3 percent by weight of a lubricant. The binder is a water-miscible aqueous dispersion. The release controlling agent functions independently of pH.

In one or more embodiments, the mesalazine is about 55 to about 65 percent by weight and is micronized material with PSD in the range of D90 less than 50 microns.

In one or more embodiments, the binder is Eudragit L30 D 55.

In one or more embodiments, the first plasticizer is triethyl citrate.

In one or more embodiments, the sugar spheres are of the size of about 425 µm to 600 µm.

In one or more embodiments, the release controlling agent is ethyl cellulose N 20.

In one or more embodiments, the pore forming agent is polyvinylpyrrolidone K-30.

In one or more embodiments, the second plasticizer is dibutyl sebacate.

In one or more embodiments, the lubricant is purified talc.

In one or more embodiments, the mesalazine extended release composition includes in vitro dissolution: upto 30 % dissolution in 1 hour; 20 %-60 % dissolution in 2 hours; 40 %-80 % dissolution in 4 hours; and 75 %-100 % dissolution in 8 hours.

In one or more embodiments, the composition is in the form of pellets packaged in a sachet.

In another embodiment, the present invention relates to a process for preparation of a mesalazine extended release composition. The process comprising: preparing a binder solution by mixing a binder and a first plasticizer; layering mesalazine using the binder solution onto sugar spheres to form drug layered pellets; preparing an extended release coating suspension by mixing a release controlling agent, a pore forming agent, a second plasticizer, and a lubricant; performing an extended release coating by applying the extended release coating suspension on to the drug layered pellets to form extended release pellets; and lubricating the extended release coated pellets.

In one or more embodiments, the process includes the drug layering is performed in a flat bottom coating pan, the extended release coating is performed in a fluid bed coater, and the lubrication is performed in a conta blender.

In one or more embodiments, the binder is Eudragit^{®} L30 D 55.

In one or more embodiments, the first plasticizer is triethyl citrate.

In one or more embodiments, the sugar spheres are of the size of about 425 µm to 600 µm.

In one or more embodiments, the release controlling agent is ethyl cellulose N 20.

In one or more embodiments, the pore forming agent is polyvinylpyrrolidone K-30 (povidone K 30).

In one or more embodiments, the second plasticizer is dibutyl sebacate.

In one or more embodiments, the lubricant is purified talc.

### BRIEF DESCRIPTION OF THE DRAWINGS

The advantages and features of the present invention will become better understood with reference to the following detailed description and claims taken in conjunction with the accompanying drawing, in which:
FIG. 1 illustrates a process flow for preparation of a mesalazine extended release composition, in accordance with an exemplary embodiment of the present invention;
FIG. 2 illustrates details of preparation of a binder solution of the process of FIG. 1;
FIG. 3 illustrates details of preparation of drug layered pellets of the process of FIG. 1;
FIG. 4 illustrates details of preparation of an extended release coating suspension of the process of FIG. 1;
FIG. 5 illustrates details for performing an extended release coating in the process of FIG. 1; and
FIG. 6 illustrates details of lubrication of extended release coated pellets in the process of FIG. 1.

### DETAILED DESCRIPTION OF THE INVENTION

The exemplary embodiments described herein detail for illustrative purposes are subject to many variations in structure and design. It should be emphasized, however, that the present invention is not limited to process for preparation of mesalazine extended release composition and mesalazine extended release composition thereof, as shown and described. It is understood that various omissions and substitutions of equivalents are contemplated as circumstances may suggest or render expedient, but these are intended to cover the application or implementation without departing from the spirit or scope of the claims of the present invention. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items. Further, the terms, "a" and "an" herein do not denote a limitation of quantity, but rather denote the presence of at least one of the referenced items.

Further, the terms, "a" and "an" herein do not denote a limitation of quantity, but rather denote the presence of at least one of the referenced items.

As used herein, "about" will be understood by persons of ordinary skill in the art and will vary to some extent depending upon the context in which it is used. If there are uses of the term in which are not clear to persons of ordinary skill in the art, given the context in which it is used, "about" will mean up to plus or minus 10 % of the particular term.

The present invention includes a patient-friendly dosing regimen for mesalazine. The Applicant discovered that these properties can be obtained by developing a sachet dosage form containing a mixture of excipients and mesalazine.

The present invention relates to a mesalazine extended release composition (in the form of pellets/sachets) and a process of preparation of mesalazine (hereinafter referred to as process). Specifically, the mesalazine extended release composition has improved extended release, requires less manufacturing time, uses minimal ingredients and has improved bioavailability. Further, all the steps of preparing the mesalazine extended release composition can be performed in minimum number of machines, thereby conducting the process of preparation with lesser number of equipments. Accordingly, the present invention provides a faster, easier, simple, robust, automated, and reproducible process for preparation of the mesalazine extended release composition.

The mesalazine extended release composition of the present invention comprises: mesalazine; sugar spheres; a binder; a first plasticizer; a release controlling agent; a pore forming agent; a second plasticizer; and a lubricant. In one embodiment the mesalazine extended release composition comprises: about 55 to about 65 percent by weight of mesalazine; about 26 to about 30 percent by weight of sugar spheres; about 4 to about 6 percent by weight of a binder; about 0.3 to about 0.75 percent by weight of a first plasticizer; about 3 to about 7 percent by weight of a release controlling agent; about 2 to about 5 percent by weight of a pore forming agent; about 0.4 to about 1.5 percent by weight of a second plasticizer; and about 1 to about 3 percent by weight of a lubricant. The binder is a water-miscible aqueous dispersion. The release controlling agent functions independently of pH.

As used herein, mesalazine, also known as mesalamine or 5-aminosalicylic acid is an anti-inflammatory drug used to treat inflammatory bowel disease, such as ulcerative colitis and mild-to-moderate Crohn's disease. Mesalazine is a bowel-specific amino salicylate drug that acts locally in the gut and has its predominant actions there, thereby having few systemic side effects. Further as used herein mesalazine pellets are the small particles created by compressing the processed mesalazine formed by the process as described below.

As used herein, sugar spheres are of the size of about 425µm to about 600µm.

"Inert core" or "core material" is the one on which mesalazine is layered to make drug particles. Herein, sugar spheres are referred as "Inert core" or "core material". The inert core according to the invention is a particle (or spherical carrier or pellet). The material used for the inert core is chemically inert, in the sense that it does not react with any of the other ingredients of the multi-layered particle according to the invention and especially does not interfere with the intended pharmacological mechanism exerted by the pharmaceutically active ingredient of the multi-layered particle. Examples for a material used for the inert core are cellulose, spheres of esp. microcrystalline cellulose, starch, lactose, sugar spheres, mannitol or mixtures thereof, or any other granular material such as granular mannitol, sugar crystals. Commercially available inert core materials can also be used. It has been found advantageous to use particles with a particle size (diameter) of about 425 µm - 600 µm.

In one embodiment, the mesalazine is about 55 to about 65 percent by weight and is micronized material with PSD in the range of D90 less than 50 microns.

Binders used in accordance with the present invention include but are not limited to tragacanth, acacia, gelatin, starch, cellulose materials such as methyl cellulose, sodium carboxy methyl cellulose, hydroxypropyl cellulose, hydroxypropyl methylcellulose, polymethacrylates, povidone, sucrose, alginic acids and salts thereof, polyethylene glycol, guar gum, polysaccharide acids, sugars, and the like. As used in the present invention, the binder is a water-miscible aqueous dispersion.

In one embodiment, the binder is Eudragit^{®} L30 D55. Eudragit^{®} L30 D55 is anionic copolymer containing free carboxylic groups in ratio of 1:1 with the ester groups.

In order to make effective drug delivery system, membrane coating of release dosage form is an effective means. Polymers when coated alone, have limited ability to form the rate controlling membrane with good mechanical properties. Addition of plasticizer is vital to the formation of a robust rate-controlling membrane. Plasticizers have the ability to interact with polymer chains, and provide desired flexibility, shock resistance, and smoothness to resultant system. Plasticized polymers are substantial in pharmaceutical technology. For example, they can be used as coating materials of dosage forms, free membranes of pharmaceutical films, polymeric membranes of transdermal system, matrix systems for drug layer formulations, or microparticles. In addition to the plasticizing effect, they also play an important role in modulating drug release profiles. Selection of a plasticizer is critical for the stability of dosage form, the processing as well as in vivo performance.

In one embodiment, the first plasticizer is triethyl citrate. Triethyl citrate as a plasticizer play an important role in diverse pharmaceutical industry by improving processability of coating polymers. Triethyl citrate is ester of citric acid, which is colorless, odorless, oily liquid generally used as hydrophilic plasticizer in pharmaceutical compositions.

As used in the present invention, the release controlling agent is ethyl cellulose with viscosity varying between 7 cPs to 50 cPs. In a preferred embodiment, the release controlling agent is ethocel N 20. Ethocel N 20 is a polymer which is inert, high purity powder with no caloric value and is virtually colorless, odorless, and tasteless. They are derived from and have the polymeric "backbone" of cellulose, a naturally occurring polymer. Ethocel N 20 is effective as controlled-release coatings, microencapsulation, granulation, and taste masking. As used in the present invention, the release controlling agent functions independently of pH.

The water-soluble pore forming materials include polymeric materials such as povidone, hydroxyl propyl methyl cellulose, hydroxyl propyl cellulose, or sugar. As used in the present invention, the water-soluble pore forming material is povidone.

In one embodiment, the pore forming agent is polyvinylpyrrolidone K-30 (povidone K-30). Povidone K-30 is also commonly known as (PVP K-30). Povidone (polyvinylpyrrolidone, PVP) is used in the pharmaceutical industry as a synthetic polymer vehicle for dispersing and suspending drugs. It has multiple uses, including as a binder for tablets and capsules, a film former for ophthalmic solutions, to aid in flavoring liquids and chewable tablets, and as an adhesive for transdermal systems. The PVP K-30 has the molecular formula of (C₆H₉NO)ₙ and appears as a white to slightly off-white powder. PVP K-30 formulations are widely used in the pharmaceutical industry due to their ability to dissolve in both water and oil solvents.

In one embodiment, the second plasticizer is dibutyl sebacate (DBS). Dibutyl sebacate is an organic chemical, consisting of esters of n-butyl alcohol and saturated dibasic acids, basically sebacic acid. Dibutyl sebacate is used in oral pharmaceutical formulations as a plasticizer for film coatings on tablets, beads, and granules. It is also used as a plasticizer in controlled-release tablets and microcapsule preparations. In particular, dibutyl sebacate (DBS) provides excellent compatibility with ethyl cellulose, and shows superior properties at low temperatures, and good oil resistivity.

In one embodiment, the lubricant is purified talc. The lubricant prevents ingredients from clumping together and from sticking to the tablet punches or capsule filling machine. Further, in pellets formulation, lubricant reduces sticking of the pellets. Lubricant also ensures that tablet formation and ejection can occur with low friction between the solid and die wall. Common minerals like talc or silica, and fats, e.g., vegetable stearin, magnesium stearate or stearic acid are the most frequently used lubricants in tablets or hard gelatin capsules. The lubricant is an agent added in small quantities to tablet and capsule formulations to improve certain processing characteristics.

In one or more embodiments, the mesalazine extended release composition includes in vitro dissolution: upto 30 % dissolution in 1 hour; 20 %-60 % dissolution in 2 hours; 40 %-80 % dissolution in 4 hours; and 75 %-100 % dissolution in 8 hours. Dissolution for 8 hours is always greater than 75%, wherein the dissolution parameters for the composition are measured in a paddle system operated at 100 RPM with 900 ml of Phosphate Buffer pH 7.5. In preferred embodiment, the mesalazine extended release composition always has greater than 75% dissolution for 8 hours.

The process of the present invention comprises the steps of: binder preparation; drug layering; extended-release coating suspension preparation; extended-release coating; and lubrication.

The process for preparation of a mesalazine extended release composition comprises: preparing a binder solution by mixing a binder and a first plasticizer; layering mesalazine using the binder solution onto sugar spheres to form drug layered pellets; preparing an extended release coating suspension by mixing a release controlling agent, a pore forming agent, a second plasticizer, and a lubricant; performing an extended release coating by applying the extended release coating suspension on to the drug layered pellets to form extended release pellets; and lubricating the extended release coated pellets.

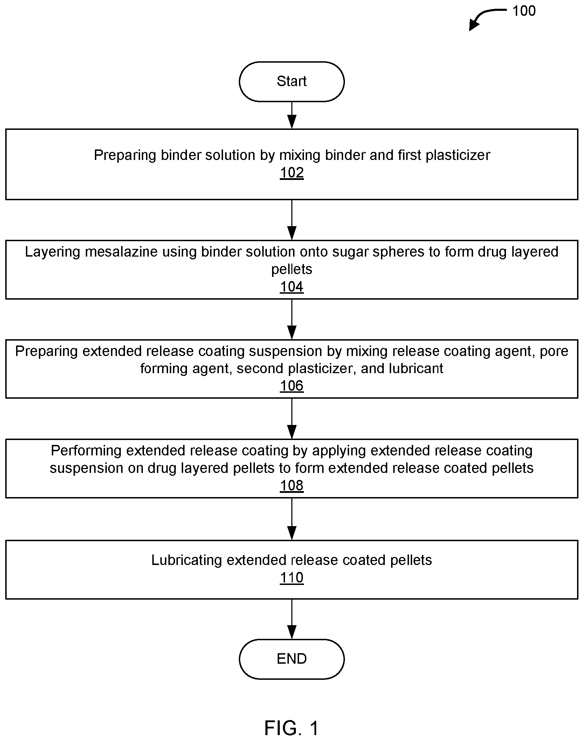

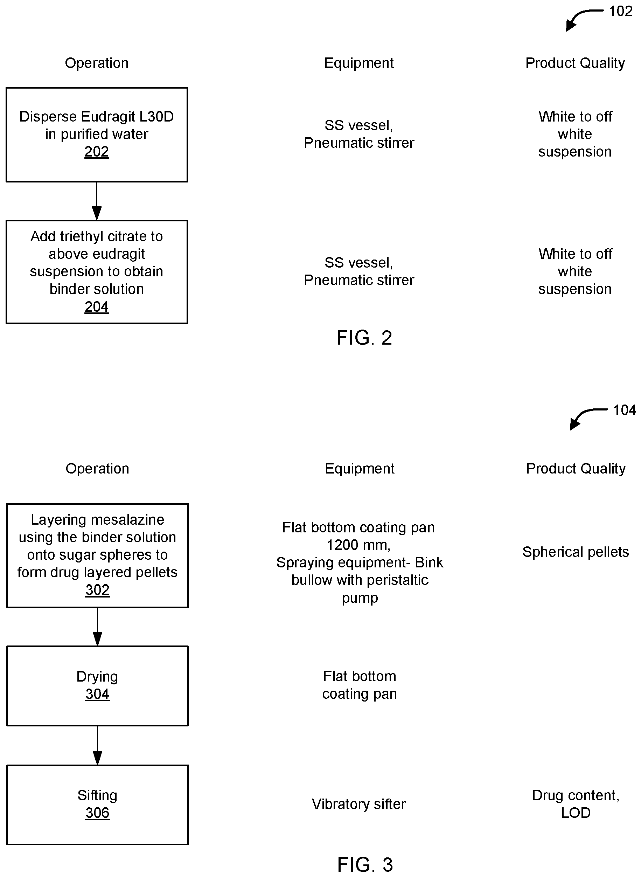

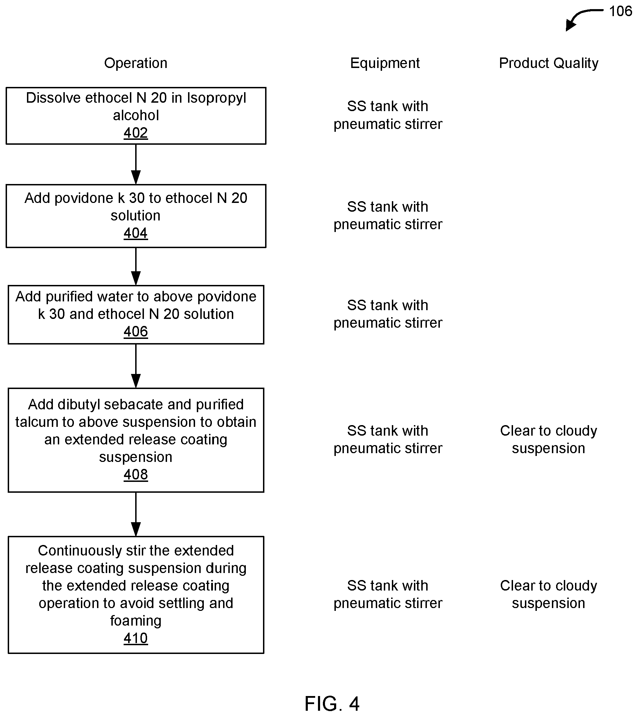

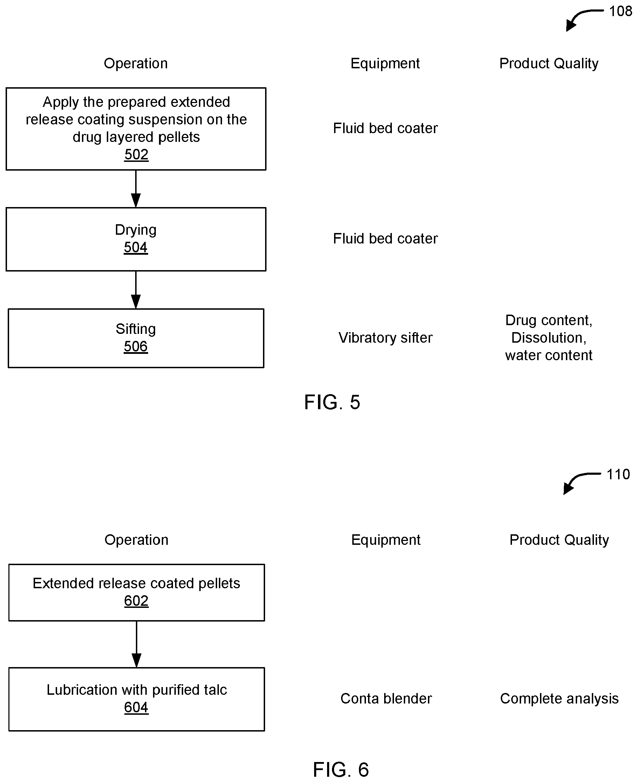

Referring to FIG. 1, at step 102, the process 100 comprises preparing a binder solution by mixing a binder and a first plasticizer. At step 104, layering mesalazine using the binder solution onto sugar spheres to form drug layered pellets. At step 106, preparing an extended release coating suspension by mixing a release controlling agent, a pore forming agent, a second plasticizer, and a lubricant. At step 108, performing an extended release coating by applying the extended release coating suspension on to the drug layered pellets to form extended release pellets. At step 110, lubricating the extended release coated pellets. The step 102 of FIG. 1 is described in detail in FIG. 2. The step 104 of FIG. 1 is described in detail in FIG. 3. The step 106 of FIG. 1 is described in detail in FIG. 4. The step 108 of FIG. 1 is described in FIG. 5. The step 110 of FIG. 1 is described in detail in FIG. 6.

Referring to FIG. 1, at step 102, the process 100 comprises preparing a binder solution comprising Eudragit^{®} L30 D55 and triethyl citrate. At step 102, preparation of the binder solution is initiated by dispersing Eudragit^{®} L30 D55 (that is, a binder) in purified water to obtain eudragit suspension. Thereafter, triethyl citrate is added to eudragit suspension to obtain binder solution. The step 102 of FIG. 1 is described in detail in FIG. 2. Referring to FIG. 2, at step 202, Eudragit^{®} L30 D55 is dispersed in purified water in SS vessel with the help of pneumatic stirrer to stir the mixture for 15 minutes to get white to off white suspension. The obtained white to off white suspension is eudragit suspension.

Next at step 204, triethyl citrate (that is, first plasticizer) is added to the eudragit suspension which is obtained at step 202. After the end of addition, stir the mixture for 15 minutes in the SS vessel with the help of the pneumatic stirrer. Thereafter, at the end of step 204, white to off white cloudy suspension is obtained. The obtained cloudy suspension is a binder solution.

Again, referring to FIG. 1, at step 104, the next step in the process 100 comprises layering mesalazine using the binder solution onto sugar spheres to form drug layered pellets. Referring to FIG. 3, at step 302, mesalazine is layered using the binder solution onto sugar spheres to form drug layered pellets in flat bottom coating pan. Specifically, the binder solution is sprayed continuously onto sugar spheres with the help of peristaltic pump of bink bullow and revolving coating pan with coating pan speed of 12-25 RPM. Mesalazine is intermittently added in the coating pan and mixed well to distribute it and adhere to sugar spheres. As a result of layering of mesalazine onto sugar spheres at step 302, drug layered pellets are obtained.

Once the entire mesalazine is adhered on the sugar spheres and drug layered pellets are obtained then at step 304, the drug layered pellets are dried in flat bottom coating pan with inlet temperature of 48-52°C and coating pan speed of 4-6 RPM. Thereafter at step 306, the previously dried drug layered pellets are sifted through 16, 18 and 20 mesh screen fitted on to vibratory sifter and agglomerate pellets are removed. Resift the drug layered pellets through 30 and 40 mesh screen fitted on the vibratory sifter and the fine pellets are removed. Collect the sifted pellets (below 16, 18 and 20 mesh screen) in HDPE container lined with polybag and the sifted pellets are weighed.

Again, referring to FIG. 1, at step 106, the next step in the process 100 comprises preparing an extended release coating suspension by mixing a release controlling agent, a pore forming agent, a second plasticizer, and a lubricant. Specifically, the extended release coating suspension is prepared by mixing ethyl cellulose N 20, purified talc, polyvinylpyrrolidone K-30, and dibutyl sebacate. The step 106 of FIG. 1 is described in detail in FIG. 4. Referring to FIG. 4, at step 402, ethocel N 20 is added to isopropyl alcohol in SS tank fitted with pneumatic stirrer and continued stirring process for 30 minutes to dissolve the mixture completely. Thereafter, at step 404, povidone K 30 is added into the above solution obtained at step 402 under stirring and dissolve by continuous stirring process for 15 minutes to form povidone K 30 and ethocel N 20 solution. Thereafter, at step 406, purified water is added to the above solution obtained at step 404 under stirring and continued stirring process for 10 minutes to form povidone K 30 and ethocel N 20 suspension. Thereafter, at step 408, dibutyl sebacate and purified talc are added into the above suspension obtained at step 406 under stirring and dissolved by continuous stirring process for 30 minutes to obtain clear to cloudy suspension. The obtained clear to cloudy suspension is an extended release coating suspension. Thereafter, at step 410, the extended release coating suspension obtained at step 408 is continuously stirred to avoid settling and foaming before coating on the drug layered pellets.

Again, referring to FIG. 1, at step 108, the next step in the process 100 comprises performing an extended release coating by applying the extended release coating suspension on to the drug layered pellets to form extended release coated pellets. The step 108 of FIG. 1 is described in detail in FIG. 5. Referring to FIG. 4 and FIG. 5, at step 502, the prepared extended release coating suspension obtained at step 410 in FIG. 4 is applied on to the drug layered pellets in a fluid bed coater at pre-determined parameters. As used herein, a non-limiting example, pre-determined parameters include an inlet temperature of about 30°C to about 50°C, product temperature of about 25°C to about 35°C and atmospheric pressure of about 1.0 kg/cm² to about 3.0 kg/cm² to obtain extended release coated pellets. Although, particularly predetermined parameters are mentioned above, it will be evident to a person skilled in the art to modify predetermined parameters based on obvious variations.

Next, at step 504, the extended release coated pellets are dried in the fluid bed coater at the pre-determined parameters. As used herein, in a non-limiting example, predetermined parameters include an inlet temperature of about 30°C to about 50°C, product temperature of about 25°C to about 35°C. Although particularly predetermined parameters are mentioned above, it will be evident to a person skilled in the art to modify predetermined parameters based on obvious variations. Thereafter, at step 506, the dried drug pellets are sifted and sieved through 16 and 30 mesh screen fitted on the vibratory sifter and the fine pellets are removed. The sifted pellets (below 16 mesh) are collected in HDPE container lined with polybag and the sifted pellets are weighed. Finally, after sifting process extended release coated pellets are obtained.

Now referring again to FIG. 1, at step 110, the next step in the process 100 comprises lubricating the extended release coated pellets. The step 110 of FIG. 1 is described in detail in FIG. 6. Next, at step 602, the extended release coated pellets are lubricated using purified talc in a conta blender bin and by mixing the extended release coated pellets about 5 minutes at 8 RPM to form mesalazine lubricated pellets. Thereafter, the mesalazine lubricated pellets are unloaded into suitable drums labeled with product name, batch number, and weights and transfer to pellets storage area. Finally, the calculated quantity of mesalazine lubricated pellets is filled in sachets using sachet filling machine.

Conventional instruments like flat bottom coating pan, bink bullows spray gun with nozzle, fluid bed coater, pneumatic stirrer, conta blender, sachet filing machine are used for the manufacturing of 1 g and 2 g mesalazine drug pellets.

The description of the present invention of pharmaceutical composition and process for preparation thereof is further illustrated by the following non-limiting examples. A person skilled in the art would recognize that, the specific examples are intended to illustrate, not limit, the scope of the present invention.

### EXAMPLES

### Drug Layering

In drug layering, a process for preparing a mesalazine extended release composition (mesalazine pellets) is described.

In first batch, the process for preparing mesalazine pellets is initiated by preparing a binder solution. The binder solution comprises Eudragit^{®} L30 D55 and triethyl citrate. 505.2 g of Eudragit^{®} L30 D55 is dispersed into Quantum satis (Q. S.) purified water in the SS vessel with the help of a pneumatic stirrer to obtain white to off white suspension. The white to off white suspension obtained is eudragit suspension. Thereafter, 15.3 g of triethyl citrate is added to the eudragit suspension. After end of the addition, stir the mixture in the SS vessel with the help of the pneumatic stirrer. Thereafter, white to off white cloudy suspension is obtained. The obtained cloudy suspension is a binder solution.

The next step is layering mesalazine onto sugar spheres by using the binder solution to form drug layered pellets (spherical drug pellets) in a flat bottom coating pan. Specifically, the binder solution is sprayed continuously on 880.38 g of sugar spheres (that is, the inert core) with the help of peristaltic pump of bink bullow and revolving coating pan. 1991.81 g of mesalazine is intermittently added in the coating pan and mixed well to distribute it and adhere to the sugar spheres.

Once the entire mesalazine is adhered on the sugar spheres, then the sugar spheres are dried in the revolving coating pan (with inlet temperature of 48-52°C) in hot air to obtain dried drug layered pellets. Then, the drug layered pellets are sifted through the screen fitted onto the vibratory sifter and agglomerate pellets are removed. The drug pellets are resifted through the screen fitted on the vibratory sifter and the fine pellets are removed. Collect the sifted pellets in HDPE container lined with polybag and the sifted pellets are weighed. In one embodiment, the layering or coating is done in a flat bottom coating pan or in a fluid bed coater or using extrusion and spheronisation.

In TABLE 1A below, the different ingredients for preparation of drug layered pellets which includes binder solution preparation and drug layering, are depicted. Further, TABLE 1B below shows properties of the drug layered pellets.

**TABLE 1A**

| Ingredients | Solution | Qty. (g) |
|---|---|---|
| Mesalazine (2% extra Active Pharmaceutical Ingredient (API) to compensate process loss) | Active | 1991.81 (1952.76 + 39.05) |
| Sugar spheres (425 µm - 600 µm) | Core | 880.38 |
| Eudragit^{®} L30 D55 | Binder | 505.2 (Solid - 151.56) |
| | | (% solid- 5.05%) |
| Triethyl citrate | First plasticizer | 15.3 |
| Purified water | Solvent | Q.S. |

**TABLE 1B**

| | |
|---|---|
| % Yield | 97.8% |
| Theoretical Assay | 650.92 mg/g |
| Practical Assay | 645.71 mg/g |

In second batch, the process for preparing mesalazine pellets is initiated by preparing a binder solution. The binder solution comprises Eudragit^{®} L30 D55 and triethyl citrate. 842.0 g of Eudragit^{®} L30 D55 is dispersed into Quantum satis (Q.S.) purified water in the SS vessel with the help of a pneumatic stirrer to obtain white to off white suspension. The white to off white suspension obtained is eudragit suspension. Thereafter, 25.5 g of triethyl citrate is added to the eudragit suspension. After end of the addition, stir the mixture in the SS vessel with the help of the pneumatic stirrer. Thereafter, white to off white cloudy suspension is obtained. The obtained cloudy suspension is a binder solution.

The next step is layering mesalazine onto sugar spheres by using the binder solution to form drug layered pellets (spherical drug pellets) in a flat bottom coating pan. Specifically, the binder solution is sprayed continuously on 1467.3 g of sugar spheres (that is, the inert core) with the help of peristaltic pump of bink bullow and revolving coating pan. 3319.69 g of mesalazine is intermittently added in the coating pan and mixed well to distribute it and adhere to the sugar spheres.

Once the entire mesalazine is adhered on the sugar spheres, then the sugar spheres are dried in the revolving coating pan (with inlet temperature of 48-52°C) in hot air to obtain dried drug layered pellets. Then, the drug layered pellets are sifted through the screen fitted onto the vibratory sifter and agglomerate pellets are removed. The drug pellets are resifted through the screen fitted on the vibratory sifter and the fine pellets are removed. Collect the sifted pellets in HDPE container lined with polybag and the sifted pellets are weighed. In one embodiment, the layering or coating is done in a flat bottom coating pan or in a fluid bed coater or using extrusion and spheronisation.

In TABLE 2A below, the different ingredients for preparation of drug layered pellets which includes binder solution preparation and drug layering, are depicted. Further, TABLE 2B below shows properties of the drug layered pellets.

**TABLE 2A**

| Ingredients | Solution | Qty. (g) |
|---|---|---|
| Mesalazine (2% extra Active Pharmaceutical Ingredient (API) to compensate process loss) | Active | 3319.69 (3254.6 + 65.09) |
| Sugar spheres (425 µm - 600 µm) | Core | 1467.3 |
| Eudragit^{®} L30 D55 | Binder | 842.0 (Solid - 252.6) |
| | | (% Solid - 5.05%) |
| Triethyl citrate | First plasticizer | 25.5 |
| Purified water | Solvent | Q.S. |

**TABLE 2B**

| | |
|---|---|
| % Yield | 97.92% |
| Theoretical Assay | 650.92 mg/g |
| Practical Assay | 644.41 mg/g |

### Extended Release Coating

In extended release coating, a process for preparing mesalazine extended release coated pellets is described.

In first batch, the extended release coating suspension is prepared by mixing a release controlling agent, a pore forming agent, a second plasticizer, and a lubricant. Specifically, the extended release coating suspension is prepared by mixing ethyl cellulose N 20, polyvinylpyrrolidone K-30, dibutyl sebacate, and purified talc. SS tank with pneumatic stirrer is taken in which Q.S. isopropyl alcohol is transferred and thereafter 29.19 g of ethyl cellulose N 20 (Ethocel N 20) is added and stirred to mix the solution well. 19.76 g of povidone K 30 is added to the solution made by the above steps to form povidone K 30 and ethocel N 20 solution. Thereafter, Q.S. purified water is added to the above obtained solution under stirring and continued stirring to form povidone K 30 and ethocel N 20 suspension. Thereafter, 5.29 g of dibutyl sebacate and 10.76 g of purified talc are added to the povidone K 30 and ethocel N 20 suspension under stirring and dissolved by continuous stirring process to obtain extended release coating suspension. Further, the obtained extended release coating suspension is continuously stirred to avoid settling and foaming before coating on drug pellets.

Next, an extended release coating is performed. The extended release coating is performed by applying the prepared extended release coating suspension on the drug layered pellets to form extended release coated pellets. Specifically, the prepared extended release coating suspension is applied on to the drug layered pellets in a fluid bed coater at pre-determined parameters. As used herein, a non-limiting example, pre-determined parameters include an inlet temperature of about 30°C to about 50°C, product temperature of about 25°C to about 35°C to obtain extended release coated pellets. Although, particular predetermined parameters are mentioned above, it will be evident to a person skilled in the art to modify predetermined parameters based on obvious variations.

Thereafter, the extended release coated pellets are dried in the fluidized bed coater at the pre-determined parameters. As used herein, in a non-limiting example, predetermined parameters include an inlet temperature of about 30°C to about 50°C, product temperature of about 25°C to about 35°C. Although particularly pre-determined parameters are mentioned above, it will be evident to a person skilled in the art to modify predetermined parameters based on obvious variations. Thereafter, the extended release coated pellets are sifted and sieved through the screen fitted on vibratory sifter and the fine pellets are removed. The sifted pellets are collected in HDPE container lined with polybag and the sifted pellets are weighed. Finally, after sifting process extended release coated pellets are obtained.

In TABLE 3A, the different ingredients for preparing extended release coating which includes preparation of extended release coating suspension and performing the extended release coating, are depicted. Further, TABLE 3B below shows properties of the extended release coated pellets. Further, TABLE 3C below shows dissolution data of the extended release coated pellets.

**TABLE 3A**

| Ingredients | Solution | Qty. (g) |
|---|---|---|
| Mesalazine drug pellets | --- | 500 |
| Ethyl cellulose N 20 (Ethocel N 20) | Release controlling agent | 29.19 |
| Polyvinylpyrrolidone K-30 (Povidone K 30) | Pore forming agent | 19.76 |
| Dibutyl sebacate | Second plasticizer | 5.29 |
| Purified Water | Solvent | Q.S. |
| Purified Talcum | Lubricant | 10.76 |
| Isopropyl alcohol | Solvent | Q.S. |

**TABLE 3B**

| | |
|---|---|
| % Yield | 97.17% |
| Product bed temperature | 27 - 31°C |
| LOD (Limit of Detection) | 1.58 % |
| Theoretical Assay | 576.03 mg/g |
| Practical Assay | 572.57 mg/g |

**TABLE 3C**

| | | |
|---|---|---|
| Dissolution (Paddle 100 RPM, 900 ml of Phosphate buffer pH 7.5) | 1 hour: Not more than 30 % | 17 % |
| | 2 hours: 20 % - 60 % | 43 % |
| | 4 hours: 40 % - 80 % | 68 % |
| | 8 hours: Not less than 75 % | 97 % |

In second batch, the extended release coating suspension is prepared by mixing a release controlling agent, a pore forming agent, a second plasticizer, and a lubricant. Specifically, the extended release coating suspension is prepared by mixing ethyl cellulose N 20, polyvinylpyrrolidone K-30, dibutyl sebacate, and purified talc. SS tank with pneumatic stirrer is taken in which Q.S. isopropyl alcohol is transferred and thereafter 53.89 g of ethyl cellulose N 20 (Ethocel N 20) is added and stirred to mix the solution well. 36.47 g of povidone K 30 is added to the solution made by the above steps to form povidone K 30 and ethocel N 20 solution. Thereafter, Q.S. purified water is added to the above obtained solution under stirring and continued stirring to form povidone K 30 and ethocel N 20 suspension. Thereafter, 9.77 g of dibutyl sebacate and 19.86 g of purified talc are added to the povidone K 30 and ethocel N 20 suspension under stirring and dissolved by continuous stirring process to obtain extended release coating suspension. Further, the obtained extended release coating suspension is continuously stirred to avoid settling and foaming before coating on drug pellets.

Next, an extended release coating is performed. The extended release coating is performed by applying the prepared extended release coating suspension on the drug layered pellets to form extended release coated pellets. Specifically, the prepared extended release coating suspension is applied on to the drug layered pellets in a fluid bed coater at pre-determined parameters. As used herein, a non-limiting example, pre-determined parameters include an inlet temperature of about 30°C to about 50°C, product temperature of about 25°C to about 35°C to obtain extended release coated pellets. Although, particular predetermined parameters are mentioned above, it will be evident to a person skilled in the art to modify predetermined parameters based on obvious variations.

Thereafter, the extended release coated pellets are dried in the fluidized bed coater at the pre-determined parameters. As used herein, in a non-limiting example, predetermined parameters include an inlet temperature of about 30°C to about 50°C, product temperature of about 25°C to about 35°C. Although particularly pre-determined parameters are mentioned above, it will be evident to a person skilled in the art to modify predetermined parameters based on obvious variations. Thereafter, the extended release coated pellets are sifted and sieved through the screen fitted on vibratory sifter and the fine pellets are removed. The sifted pellets are collected in HDPE container lined with polybag and the sifted pellets are weighed. Finally, after sifting process extended release coated pellets are obtained.

In TABLE 4A, the different ingredients for preparing extended release coating which includes preparation of extended release coating suspension and performing the extended release coating, are depicted. Further, TABLE 4B below shows properties of the extended release coated pellets. Further, TABLE 4C below shows dissolution data of the extended release coated pellets.

**TABLE 4A**

| Ingredients | Solution | Qty. (g) |
|---|---|---|
| Mesalazine drug pellets | --- | 1500 |
| Ethyl cellulose N 20 (Ethocel N 20) | Release controlling agent | 53.89 |
| Polyvinylpyrrolidone K-30 (Povidone K 30) | Pore forming agent | 36.47 |
| Dibutyl sebacate | Second plasticizer | 9.77 |
| Purified Water | Solvent | Q.S. |
| Purified Talcum | Lubricant | 19.86 |
| Isopropyl alcohol | Solvent | Q.S. |

**TABLE 4B**

| | |
|---|---|
| % Yield | 97.90% |
| Product bed temperature | 27 - 32°C |
| LOD (Limit of Detection) | 1.61 % |
| Theoretical Assay | 602.70 mg/g |
| Practical Assay | 597.3 mg/g |

**TABLE 4C**

| | | |
|---|---|---|
| Dissolution (Paddle 100 RPM, 900 ml of Phosphate buffer pH 7.5) | 1 hour: Not more than 30 % | 20 % |
| | 2 hours: 20 % - 60 % | 49 % |
| | 4 hours: 40 % - 80 % | 70 % |
| | 8 hours: Not less than 75 % | 97 % |

In third batch, the extended release coating suspension is prepared by mixing a release controlling agent, a pore forming agent, a second plasticizer, and a lubricant. Specifically, the extended release coating suspension is prepared by mixing ethyl cellulose N 20, polyvinylpyrrolidone K-30, dibutyl sebacate, and purified talc. SS tank with pneumatic stirrer is taken in which Q.S. isopropyl alcohol is transferred and thereafter 53.89 g of ethyl cellulose N 20 (Ethocel N 20) is added and stirred to mix the solution well. 36.47 g of povidone K 30 is added to the solution made by the above steps to form povidone K 30 and ethocel N 20 solution. Thereafter, Q.S. purified water is added to the above obtained solution under stirring and continued stirring to form povidone K 30 and ethocel N 20 suspension. Thereafter, 9.77 g of dibutyl sebacate and 19.86 g of purified talc are added to the povidone K 30 and ethocel N 20 suspension under stirring and dissolved by continuous stirring process to obtain extended release coating suspension. Further, the obtained extended release coating suspension is continuously stirred to avoid settling and foaming before coating on drug pellets.

Next, an extended release coating is performed. The extended release coating is performed by applying the prepared extended release coating suspension on the drug layered pellets to form extended release coated pellets. Specifically, the prepared extended release coating suspension is applied on to the drug layered pellets in a fluid bed coater at pre-determined parameters. As used herein, a non-limiting example, pre-determined parameters include an inlet temperature of about 30°C to about 50°C, product temperature of about 25°C to about 35°C to obtain extended release coated pellets. Although, particular predetermined parameters are mentioned above, it will be evident to a person skilled in the art to modify predetermined parameters based on obvious variations.

Thereafter, the extended release coated pellets are dried in the fluidized bed coater at the pre-determined parameters. As used herein, in a non-limiting example, predetermined parameters include an inlet temperature of about 30°C to about 50°C, product temperature of about 25°C to about 35°C. Although particularly predetermined parameters are mentioned above, it will be evident to a person skilled in the art to modify predetermined parameters based on obvious variations. Thereafter, the extended release coated pellets are sifted and sieved through the screen fitted on vibratory sifter and the fine pellets are removed. The sifted pellets are collected in HDPE container lined with polybag and the sifted pellets are weighed. Finally, after sifting process extended release coated pellets are obtained.

In TABLE 5A, the different ingredients for preparing extended release coating which includes preparation of extended release coating suspension and performing the extended release coating, are depicted. Further, TABLE 5B below shows properties of the extended release coated pellets. Further, TABLE 5C below shows dissolution data of the extended release coated pellets.

**TABLE 5A**

| Ingredients | Solution | Qty. (g) |
|---|---|---|
| Mesalazine drug pellets | --- | 1500 |
| Ethyl cellulose N 20 (Ethocel N 20) | Release controlling agent | 53.89 |
| Polyvinylpyrrolidone K-30 (Povidone K 30) | Pore forming agent | 36.47 |
| Dibutyl sebacate | Second plasticizer | 9.77 |
| Purified Water | Solvent | Q.S. |
| Purified Talcum | Lubricant | 19.86 |
| Isopropyl alcohol | Solvent | Q.S. |

**TABLE 5B**

| | |
|---|---|
| % Yield | 98.21 % |
| Product bed temperature | 27 - 32°C |
| LOD (Limit of Detection) | 1.48 % |
| Theoretical Assay | 602.70 mg/g |
| Practical Assay | 599.69 mg/g |

**TABLE 5C**

| | | |
|---|---|---|
| Dissolution (Paddle 100 RPM, 900 ml of Phosphate buffer pH 7.5) | 1 hour: Not more than 30 % | 21 % |
| | 2 hours: 20 % - 60 % | 45 % |
| | 4 hours: 40 % - 80 % | 76 % |
| | 8 hours: Not less than 75 % | 95 % |

### Lubrication

In first batch, the extended release coated pellets are lubricated. 565 g of extended release coated pellets are lubricated with 2.54 g of purified talc in a conta blender bin to form mesalazine lubricated pellets.

In TABLE 6, the different ingredients for lubricating the extended release coated pellets with purified talc are depicted.

**TABLE 6**

| Ingredients | Solution | Qty. (g) |
|---|---|---|
| Extended release coated pellets | --- | 565 |
| Purified talc | Lubricant | 2.54 |

In second batch, the extended release coated pellets are lubricated. 1620 g of extended release coated pellets are lubricated with 7.29 g of purified talc in a conta blender bin to form mesalazine lubricated pellets.

In TABLE 7, the different ingredients for lubricating the extended release coated pellets with purified talc are depicted.

**TABLE 7**

| Ingredients | Solution | Qty. (g) |
|---|---|---|
| Extended release coated pellets | --- | 1620 |
| Purified talc | Lubricant | 7.29 |

In third batch, the extended release coated pellets are lubricated. 1620 g of extended release coated pellets are lubricated with 7.29 g of purified talc in a conta blender bin to form mesalazine lubricated pellets.

In TABLE 8, the different ingredients for lubricating the extended release coated pellets with purified talc are depicted.

**TABLE 8**

| Ingredients | Solution | Qty. (g) |
|---|---|---|
| Extended release coated pellets | --- | 1620 |
| Purified talc | Lubricant | 7.29 |

All the process for manufacturing mesalazine pellets as described in the above examples were done in the fluid bed coater, flat bottom coating pan and conta blender.

In one embodiment, TABLE 9 provides a percentage by weight of the quantitative composition of mesalazine extended release composition 1 g and 2 g.

**TABLE 9**

| Ingredients | Relative Qty. |
|---|---|
| | % w/w |
| Mesalazine | 60.0 |
| Sugar spheres (425 µm - 600 µm) | 27.05 |
| Eudragit^{®} L30 D55 | 4.66 |
| Triethyl Citrate | 0.47 |
| Ethyl cellulose N20 (Ethocel N 20) | 3.31 |
| Poly vinyl pyrrolidone K-30 (Povidone K 30) | 2.24 |
| Dibutyl sebacate | 0.60 |
| Purified Talc | 1.67 |
| Isopropyl alcohol | Q.S. |
| Purified Water | Q.S. |
| Total | 100.0 |

In TABLE 10, the analytical results of 1 g sachet of mesalazine extended release pellets are depicted for first batch. The specification details are: description- light to dark beige mesalazine extended release pellets filled in heat sealed aluminium sachet, Average filled weight - 1666.66 mg + 7.5 %, Water content - NMT (Not More Than) 5.0 %, Mean assay - 1.0 g/sachet (0.95 g/sachet - 1.05 g/sachet), Mean assay percentage -95.0 % - 105.0 % of label claim.

**TABLE 10**

| Description | | Dark beige spherical pellets filled in heat sealed aluminium sachet |
|---|---|---|
| Dissolution (Paddle 100 RPM, 900 ml of Phosphate buffer pH 7.5) | 1 hour (NMT 30 %) | 22 % |
| | 2 hours (20 % - 60 %) | 45 % |
| | 4 hours (40 % - 80 %) | 73 % |
| | 8 hours (NLT 75 %) | 98 % |
| Average filled weight | 1662.6 mg | |
| Water content | 2.13 % | |
| Mean assay | 0.986 g/sachet | |
| Mean assay percentage | 98.7 % | |

In TABLE 11, the analytical results of 1 g sachet of mesalazine extended release pellets are depicted for second batch. The specification details are: description- light to dark beige mesalazine extended release pellets filled in heat sealed aluminium sachet, Average filled weight - 1666.66 mg + 7.5 %, Water content - NMT (Not More Than) 5.0 %, Mean assay - 1.0 g/sachet (0.95 g/sachet - 1.05 g/sachet), Mean assay percentage -95.0 % - 105.0 % of label claim.

**TABLE 11**

| Description | | Dark beige spherical pellets filled in heat sealed aluminium sachet |
|---|---|---|
| Dissolution (Paddle 100 RPM, 900 ml of Phosphate buffer pH 7.5) | 1 hour (NMT 30 %) | 23 % |
| | 2 hours (20 % - 60 %) | 46% |
| | 4 hours (40%-80%) | 75 % |
| | 8 hours (NLT 75 %) | 99 % |
| Average filled weight | 1704.2 mg | |
| Water content | 2.17 % | |
| Mean assay | 0.983 g/sachet | |
| Mean assay percentage | 98.3 % | |

In TABLE 12, the analytical results of 1 g sachet of mesalazine extended release pellets are depicted for third batch. The specification details are: description- light to dark beige mesalazine extended release pellets filled in heat sealed aluminium sachet, Average filled weight - 1666.66 mg + 7.5 %, Water content - NMT (Not More Than) 5.0 %, Mean assay - 1.0 g/sachet (0.95 g/sachet - 1.05 g/sachet), Mean assay percentage -95.0 % - 105.0 % of label claim.

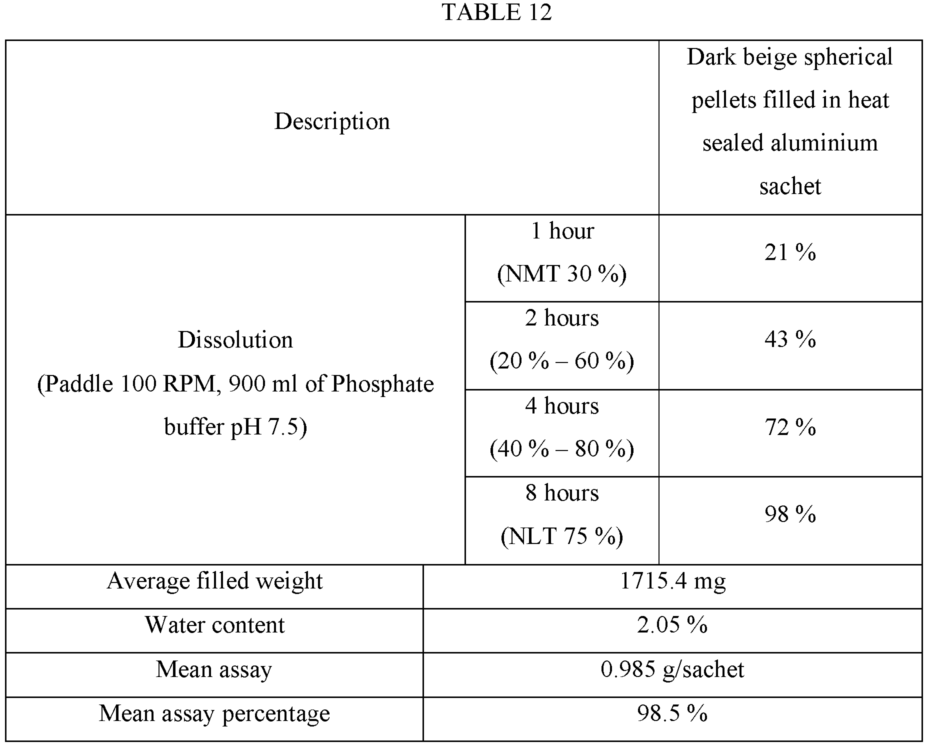

In TABLE 13, the analytical results of 2 g sachet of mesalazine extended release pellets are depicted for first batch. The specification details are: description- light to dark beige mesalazine extended release pellets filled in heat sealed aluminium sachet, Average filled weight- 3333.33 mg + 7.5 %, Water content- NMT 5.0 %, Mean assay- 2.0 g/sachet (1.90 g/sachet - 2.10 g/sachet), Mean assay percentage- 95.0 % - 105.0 % of label claim.

**TABLE 13**

| Description | | Dark beige spherical pellets filled in heat sealed aluminium sachet |
|---|---|---|
| Dissolution (Paddle 100 RPM, 900 ml of Phosphate buffer pH 7.5) | 1 hour (NMT 30 %) | 23 % |
| | 2 hours (20 % - 60 %) | 45 % |
| | 4 hours (40 % - 80 %) | 74% |
| | 8 hours (NLT 75 %) | 100 % |
| Average filled weight | 3423.7 mg | |
| Water content | 2.02 % | |
| Mean assay | 1.967 g/sachet | |
| Mean assay percentage | 98.4 % | |

In TABLE 14, the analytical results of 2 g sachet of mesalazine extended release pellets are depicted for second batch. The specification details are: description- light to dark beige mesalazine extended release pellets filled in heat sealed aluminium sachet, Average filled weight- 3333.33 mg + 7.5 %, Water content- NMT 5.0 %, Mean assay- 2.0 g/sachet (1.90 g/sachet - 2.10 g/sachet), Mean assay percentage- 95.0 % - 105.0 % of label claim.

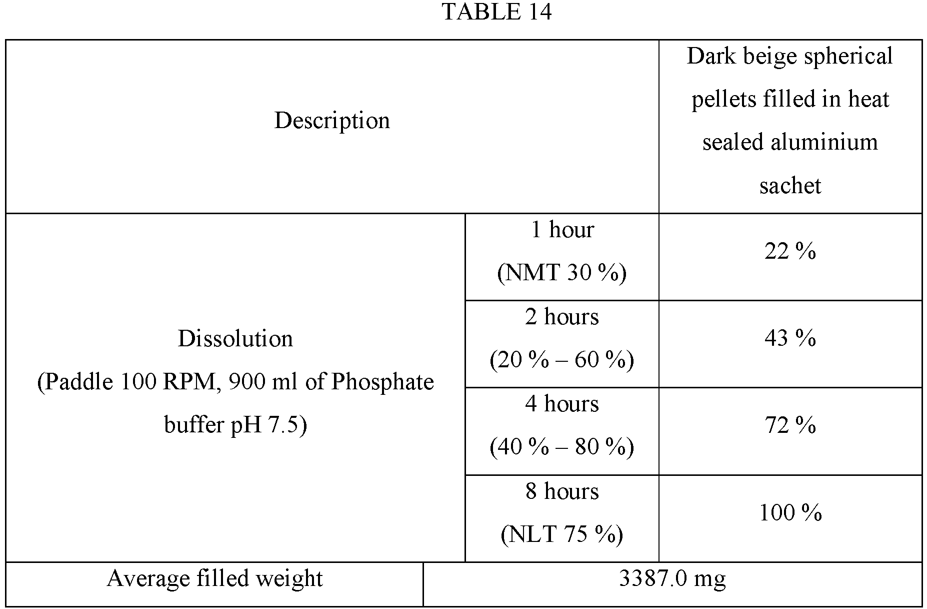

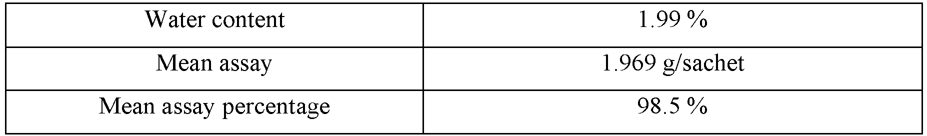

In TABLE 15, the analytical results of 2 g sachet of mesalazine extended release pellets are depicted for third batch. The specification details are: description- light to dark beige mesalazine extended release pellets filled in heat sealed aluminium sachet, Average filled weight- 3333.33 mg + 7.5 %, Water content- NMT 5.0 %, Mean assay- 2.0 g/sachet (1.90 g/sachet - 2.10 g/sachet), Mean assay percentage- 95.0 % - 105.0 % of label claim.

**TABLE 15**

| Description | | Dark beige spherical pellets filled in heat sealed aluminium sachet |
|---|---|---|
| Dissolution (Paddle 100 RPM, 900 ml of Phosphate buffer pH 7.5) | 1 hour (NMT 30 %) | 22 % |
| | 2 hours (20% - 60 %) | 44 % |
| | 4 hours (40 % - 80 %) | 73 % |
| | 8 hours (NLT 75 %) | 98 % |
| Average filled weight | 3384.9 mg | |
| Water content | 1.96 % | |
| Mean assay | 1.967 g/sachet | |
| Mean assay percentage | 98.4 % | |

The present invention provides a mesalazine extended release composition that has improved extended release pellets, requires less manufacturing time, and uses minimal ingredients. Further, the present invention provides a process for preparation of mesalazine extended release composition that is faster, simple, easier, robust, automated, and reproducible, wherein all the steps of preparation of the mesalazine extended release composition can be performed in minimum number of equipments, thereby conducting the process of preparation with lesser number of equipments/machines.

The foregoing descriptions of specific embodiments of the present disclosure have been presented for purposes of illustration and description. They are not intended to be exhaustive or to limit the present disclosure to the precise forms disclosed, and obviously many modifications and variations are possible in light of the above teaching. The embodiments were chosen and described in order to best explain the principles of the present disclosure and its practical application, to thereby enable others skilled in the art to best utilize the present disclosure and various embodiments with various modifications as are suited to the particular use contemplated. (It is understood that various omission and substitutions of equivalents are contemplated as circumstance may suggest or render expedient, but such are intended to cover the application or implementation without departing from the spirit or scope of the claims of the present disclosure).

## Claims

1. A mesalazine extended release composition, comprising:
about 55 to about 65 percent by weight of mesalazine;
about 26 to about 30 percent by weight of sugar spheres;
about 4 to about 6 percent by weight of a binder, wherein the binder is a water-miscible aqueous dispersion;
about 0.3 to about 0.75 percent by weight of a first plasticizer;
about 3 to about 7 percent by weight of a release controlling agent, wherein the release controlling agent functions independently of pH;
about 2 to about 5 percent by weight of a pore forming agent;
about 0.4 to about 1.5 percent by weight of a second plasticizer; and
about 1 to about 3 percent by weight of a lubricant.

2. The mesalazine extended release composition as claimed in claim 1, wherein the mesalazine is about 55 to about 65 percent by weight and is micronized material with PSD in the range of D90 less than 50 microns.

3. The mesalazine extended release composition as claimed in claim 1, wherein the binder is Eudragit^{®} L30 D 55.

4. The mesalazine extended release composition as claimed in claim 1, wherein the first plasticizer is triethyl citrate.

5. The mesalazine extended release composition as claimed in claim 1, wherein the sugar spheres are of the size of about 425 µm to 600 µm.

6. The mesalazine extended release composition as claimed in claim 1, wherein the release controlling agent is ethyl cellulose N 20.

7. The mesalazine extended release composition as claimed in claim 1, wherein the pore forming agent is polyvinylpyrrolidone K-30.

8. The mesalazine extended release composition as claimed in claim 1, wherein the second plasticizer is dibutyl sebacate.

9. The mesalazine extended release composition as claimed in claim 1, wherein the lubricant is purified talc.

10. The mesalazine extended release composition as claimed in claim 1, wherein in vitro dissolution array of mesalazine of
a) upto 30 % dissolution in 1 hour;
b) 20 %-60 % dissolution in 2 hours;
c) 40 %-80 % dissolution in 4 hours; and
d) 75 %-100 % dissolution in 8 hours.

11. The mesalazine extended release composition, wherein the composition is in the form of pellets packaged in a sachet.

12. A process for preparation of a mesalazine extended release composition, comprising;
preparing a binder solution by mixing a binder and a first plasticizer; layering mesalazine using the binder solution onto sugar spheres to form drug layered pellets;
preparing an extended release coating suspension by mixing a release controlling agent, a pore forming agent, a second plasticizer, and a lubricant;
performing an extended release coating by applying the extended release coating suspension on to the drug layered pellets to form extended release coated pellets; and
lubricating the extended release coated pellets.

13. The process as claimed in claim 12, wherein the drug layering is performed in a flat bottom coating pan, the extended release coating is performed in a fluid bed coater, and the lubrication is performed in a conta blender.

14. The process as claimed in claim 12, wherein the binder is Eudragit^{®} L30 D 55.

15. The process as claimed in claim 12, wherein the first plasticizer is triethyl citrate.

16. The process as claimed in claim 12, wherein the sugar spheres are of the size of about 425 µm to 600 µm.

17. The process as claimed in claim 12, wherein the release controlling agent is ethyl cellulose N 20.

18. The process as claimed in claim 12, wherein the pore forming agent is polyvinylpyrrolidone K-30.

19. The process as claimed in claim 12, wherein the second plasticizer is dibutyl sebacate.

20. The process as claimed in claim 12, wherein the lubricant is purified talc.
